# EUROPÄISCHE PATENTSCHRIFT

(11) **EP 2 633 887 B1**
(45) Veröffentlichungstag und Bekanntmachung des Hinweises auf die Patenterteilung: **02.03.2016**
(21) Anmeldenummer: 12189513.0
(22) Anmeldetag: 23.10.2012
(51) Int. Cl.: A61K 8/63, A61K 8/67, A61Q 19/08, A61K 8/73, A61Q 19/00, A61K 8/49, A61K 8/34, A61K 8/60

(54) **Neuartige Wirkstoffkombination zur effizienten Anti-Faltenwirkung**
Novel agent combination for efficient anti-wrinkle effect
Combinaison de principes actifs d'un nouveau type pour une action anti-plis efficace

(30) Priorität: 29.11.2011 DE 102011087320
(43) Veröffentlichungstag der Anmeldung: 04.09.2013
(73) Patentinhaber: Henkel AG&Co. KGAA, 40589 Düsseldorf (DE)
(72) Erfinder: Bösel, Tanja, 40764 Langenfeld (DE); Waldmann-Laue, Marianne, 40789 Monheim (DE); Holtkötter, Olaf, 50354 Hürth (DE)

(56) Entgegenhaltungen:
- EP-A1- 0 747 042
- EP-A2- 0 803 248
- WO-A1-01/07009
- WO-A1-2005/041955
- WO-A1-2006/053415
- WO-A1-2009/152205
- WO-A2-01/06829
- WO-A2-02/07707
- WO-A2-02/26207
- WO-A2-02/053108

## Beschreibung

Die Erfindung liegt auf dem Gebiet der Kosmetik und betrifft eine kosmetische oder dermatologische Zusammensetzung auf der Basis einer speziellen Wirkstoffkombination, sowie die kosmetische Verwendung der Zusammensetzung zur Reduktion von Hautfalten.

Die Erhaltung "junger Haut" ist seit jeher ein zentrales Ziel bei der Entwicklung kosmetischer Hautbehandlungsmittel.

Aus dem Stand der Technik ist eine Vielzahl kosmetischer Wirkstoffe und/oder Wirkstoffkombinationen bekannt, die einen sogenannten Anti-Aging-Effekt auf der Haut zeigen, doch die bereits bekannten Mittel können die stetig wachsenden Bedürfnisse der Verbraucher nicht vollständig abdecken.

Es ist beispielsweise bekannt, dass Retinol und/oder Retinoide in der Dermis die Neuproduktion von Bestandteilen der extrazellulären Matrix, beispielsweise des Kollagens, stimulieren, und in der Epidermis die Proliferation und Differenzierung der Keratinocyten regulieren kann (können). Makroskopisch führt die Verwendung von Retinsäure und/oder Retinol in topischen Zusammensetzungen zu einem verfeinerten Hautbild, einer Aufhellung von Pigment- und/oder Altersflecken, zu einer Normalisierung der Epidermisdicke sowie zu einer Straffung der Haut. Problematisch bei der Verwendung von Retinoiden - insbesondere in kosmetischen Zusammensetzungen - sind die starken Nebenwirkungen der Retinoide (Teratogenität, Hypertriglyceridaemie und/oder retinoide Dermatitis).

Diese unerwünschten Nebenwirkungen schließen eine Verwendung von Retinoiden im kosmetischen Massenmarkt nahezu aus.

In der Vergangenheit war man deshalb bemüht, wirksame Alternativen für Retinoide zu finden, die die gleichen positiven Eigenschaften wie die Retinoide aufweisen, gleichzeitig aber die zuvor genannten, negativen Eigenschaften der Retinoide nicht aufweisen.

So werden beispielsweise in der Offenlegungsschrift WO 02/26207 A2 Zusammensetzungen zur Behandlung der Hautalterung beschrieben, welche ein Azol (Imidazolynidylharnstoff), ein Tocopherol (Vitamin E acetat), Glycyrrhizinsäure, Hyaluronsäure sowie ein Polyol (Glycerol) enthalten.

In der Offenlegungsschrift EP 0 803 248 A2 werden Zusammensetzungen zur Behandlung von Anzeichen der Hautalterung offenbart, welche Retinol und/oder Retinylester, Azole sowie Fettsäureamide enthalten.

Weiterhin wird in der Offenlegungsschrift WO 2006/053415 A1 die Verwendung von Pflanzenextrakten, welche die extrazelluläre Protease inhibieren können, zur Verhinderung bzw. zur Verminderung der Hautalterung offenbart.

Die Verwendung einer Kombination von Azolen mit einem Fettmaterial, ausgewählt aus der Gruppe von Ceramiden, Pseudoceramiden, Neoceramiden sowie deren Mischungen, zur Verminderung der Hautalterung wird in der Offenlegungsschrift EP 0 747 042 A1 beschrieben.

In der Offenlegungsschrift WO 02/053108 A2 wird die Verwendung eines Hautpflegeprodukts zur Verminderung von Anzeichen der Hauthalterung beschrieben. Dieses Hautpflegeprodukt enthält in einem ersten Behälter eine Zusammensetzung mit Retinoiden und in einen zweiten Behälter eine Zusammensetzung mit einem Retinoidbooster und einem Phytoestrogen.

Die Verwendung von Imidazoldindionen zur Verminderung von Falten wird in der Offenlegungsschrift WO 2009/152205 A1 offenbart.

Weiterhin beschreibt die Offenlegungsschrift WO 02/07707 A2 die Verwendung von funktionalisierten Mikropartikeln zur Abgabe von Wirkstoffen auf die Oberfläche der Haut, der Nägel und der Haare.

Zudem beschreibt die Offenlegungsschrift WO 01/06829 A2 die Verwendung von speziellen Linkermolekülen zur kovalenten Anbindung von Wirkstoffen an Körpergewebe.

Die Verwendung von Lysinoxidasen zur kovalenten Anbindung von Wirkstoffen an Körpergewebe wird in der Offenlegungsschrift WO 01/07009 A1 beschrieben

Schließlich wird in der Offenlegungsschrift WO 2005/041955 A1 die Verwendung eines Imidazols zur Behandlung von entzündlichen Hautkrankheiten, welche nicht durch Bakterien verursacht werden, offenbart.

Der vorliegenden Erfindung lag die Aufgabe zugrunde, Wirkstoffe und/oder Wirkstoffkombinationen zu finden, die sich für die Verwendung in kosmetischen - insbesondere dermatologischen - Mitteln eignen, und die eine signifikante Antifaltenwirkung aufweisen.

Die Antifaltenwirkung der Wirkstoffe und/oder der Wirkstoffkombination sollte vergleichbar der zuvor beschrieben Wirkung der Retinoide sein, ohne die Nachteile der Retinoide aufzuweisen.

Die Antifaltenwirkung sollte schnell sichtbar und nachhaltig sein.

Weiterhin sollten die Anzeichen müder und/oder schlaffer Haut wirksam bekämpft, und die Hautfeuchtigkeit erhöht werden.

Diese Aufgaben konnten erfüllt werden durch die kosmetische Verwendung einer spezifischen Wirkstoffkombination.

Gegenstand der vorliegenden Erfindung ist demnach eine kosmetische Zusammensetzung, die in einem geeigneten kosmetischen oder dermatologischen Träger
(a) Climbazol und/oder eines seiner physiologisch verträglichen Salze,
(b) Tocopherol und/oder ein(e) physiologisch verträgliche(s) Salz,
(b) Glycyrrhizinsäure und/oder ein physiologisch verträgliches Salz,
(c) Hyaluronsäure bzw. ein physiologisch verträgliches Salz und
(d) mindestens ein Polyol enthält.

Unter einer "kosmetischen Zusammensetzung" wird bevorzugt eine Zusammensetzung für die Behandlung der Haut und/oder der Hautanhangsgebilde - besonders bevorzugt für die Behandlung der Haut - verstanden.

Hautbehandlungsmittel im Sinne der vorliegenden Erfindung können in verschiedenen Angebotsformen konfektioniert werden, beispielsweise als (ggf. öl- und fettfreies) Gel, als Creme, in Stiftform, als flüssige oder gelförmige Roll-on-Applikation, als getränktes flexibles Substrat (Pad), aber auch als Puder oder Spray.

Hautbehandlungsmittel im Sinne der vorliegenden Erfindung können in fester, halbfester, flüssiger, disperser, emulgierter, suspendierter, oder gelförmiger Form vorliegen. Weiterhin können sie als Aerosol konfektioniert sein, das heißt, sie sind in einem Druckbehälter verpackt, aus dem sie mit Hilfe eines Treibmittels versprüht werden können. Weiterhin können die Hautbehandlungsmittel als treibgasfreies Pumpspray versprüht werden.

Bevorzugte Hautbehandlungsmittel im Sinne der vorliegenden Erfindung können beispielsweise Reinigungsmittel für die Haut wie Dusch- und Waschgele und/oder Seifen, Hautpflegemittel wie Lotionen, Gele und/oder Cremes für den Körper und/oder das Gesicht, Gesichtswässer, Lippenpflegemittel, aber auch dekorative kosmetische Mittel wie Makeups sein.

Besonders bevorzugte Hautbehandlungsmittel sind dadurch gekennzeichnet, dass sie als Hautpflegemittel, insbesondere als Hautpflegemittel für die Gesichtshaut, wie eine Gesichtscreme, ein Gesichtsgel und/oder ein Gesichtswasser, konfektioniert sind.

Unter einem geeigneten kosmetischen oder dermatologischen Träger wird bevorzugt ein wässriger oder wässrig-alkoholischer Träger verstanden.

Bevorzugt enthält der Träger - bezogen auf sein Gesamtgewicht - mindestens 30 Gew.-%, mehr bevorzugt mindestens 35 Gew.-% besonders bevorzugt mindestens 40 Gew.-% und insbesondere mindestens 45 Gew.-% Wasser.

Weiterhin kann der kosmetische Träger 0,01 bis 50 Gew.-%, bevorzugt 0,05 bis 40 Gew.-% und insbesondere 0,1 bis 30 Gew.-% mindestens eines Alkohols enthalten, der ausgewählt sein kann aus Ethanol, 1-Propanol, 2-Propanol, Isopropanol, Glycerin, Diglycerin, Triglycerin, 1-Butanol, 2-Butanol, 1,2-Propylenglycol, 1,2-Butandiol, 1,3-Butandiol, 1-Pentanol, 2-Pentanol, 1,2-Pentandiol, 1,5-Pentandiol, 1, Hexanol, 2-Hexanol, 1,2-Hexandiol, 1,6-Hexandiol, Polyethylenglycole, Sorbitol, Sorbitan, Benzylalkohol, Phenoxyethanol oder Mischungen dieser Alkohole.

Bevorzugt sind die wasserlöslichen Alkohole.

Besonders bevorzugt sind Ethanol, 1-Propanol, 2-Propanol, 1,2-Propylenglycol, Glycerin, und/oder 1,6-Hexandiol sowie Mischungen dieser Alkohole. Insbesondere bevorzugt sind Glycerin und/oder 1,6-Hexandiol.

Als Bestandteil (a) wird erfindungsgemäß die unter der INCI-Bezeichnung Climbazole bekannte Verbindung, sowie deren physiologisch verträglichen Salze und/oder deren Mischungen eingesetzt, denn es wurde gefunden, dass Climbazol die die natürliche Kollagensynthese sowie die natürliche Hyaluronsäuresynthese in der extrazellulären Matrix stimulieren und/oder verbessern kann.

Das Climbazol (a) kann in der erfindungsgemäßen Zusammensetzungen bevorzugt in Mengen von 0,001 bis 2 Gew.-%, mehr bevorzugt von 0,0025 bis 1,5 Gew.-%, und insbesondere von 0,005 bis 1 Gew.-% eingesetzt werden, wobei sich die Mengenangaben auf das Gesamtgewicht der Zusammensetzung beziehen.

Besonders bevorzugt enthält eine erfindungsgemäße Zusammensetzung - bezogen auf ihr Gesamtgewicht - 0,005 bis 1 Gew.-% und insbesondere 0,01 bis 0,5 Gew.-% Climbazole.

Unter "Tocopherol und/oder einem physiologisch verträglichen Salz (b)" wird bevorzugt Tocopherol, insbesondere alpha-Tocopherol, verstanden. Bevorzugte Derivate sind insbesondere die Ester, wie Tocopherylacetat, -nicotinat, -phosphat, -succinat, -linoleat, -oleat, Tocophereth-5, Tocophereth-10, Tocophereth-12, Tocophereth-18, Tocophereth-50 und/oder Tocophersolan. Insbesondere bevorzugt enthalten die erfindungsgemäßen Zusammensetzungen alpha-Tocopherol und/oder alpha-Tocopherolacetat.

Die Komponente(n) (b) kann (können) in den erfindungsgemäßen Zusammensetzungen - bezogen auf deren Gesamtgewicht - bevorzugt in Mengen von 0,01 - 3 Gew.-%, mehr bevorzugt von 0,05 bis 2 Gew.-% und insbesondere von 0,1 bis 1 Gew.-% eingesetzt werden.

Besonders bevorzugt enthält eine erfindungsgemäße Zusammensetzung - bezogen auf ihr Gesamtgewicht - 0,05 bis 2 Gew.-%, insbesondere 0,1 bis 1 Gew.-% alpha-Tocopherol und/oder alpha-Tocopherolacetat.

Unter "Glycyrrhitinsäure (3-ß-Hydroxy-11oxoolean-12-en-30-säure) oder einem physiologisch verträglichen Salz (c)" wird bevorzugt Glycyrrhitinsäure und/oder ein Alkali- oder Ammoniumsalz der Glycyrrhitinsäure verstanden.

Besonders bevorzugt ist Glycyrrhitinsäure und/oder Ammonium Glycyrrhizate.

Die Komponente(n) (c) kann (können) in den erfindungsgemäßen Zusammensetzungen - bezogen auf deren Gesamtgewicht - bevorzugt in Mengen von 0,01 - 5 Gew.-%, mehr bevorzugt von 0,025 bis 3 Gew.-% und insbesondere von 0,05 bis 1 Gew.-% eingesetzt werden.

Besonders bevorzugt enthält eine erfindungsgemäße Zusammensetzung - bezogen auf ihr Gesamtgewicht - 0,05 bis 1 Gew.-%, insbesondere 0,1 bis 1 Gew.-% Glycyrrhitinsäure und/oder Ammonium Glycyrrhizate..

Unter "Hyaluronsäure oder einem physiologisch verträglichen Salz (d)" wird bevorzugt Hyaluronsäure und/oder ein Alkali- oder Ammoniumsalz der Hyaluronsäure verstanden. Besonders bevorzugt ist Hyaluronsäure.

Alternativ kann die Hyaluronsäure auch Bestandteil kleiner Kügelchen sein, die den erfindungsgemäßen Zusammensetzungen zugegeben werden können. Geeignete Kügelchen sind beispielsweise erhältlich unter der Handelsbezeichnung Hyaluronic Filling Spheres^{®} von der Firma BASF.

Die Komponente(n) (d) kann (können) in den erfindungsgemäßen Zusammensetzungen - bezogen auf deren Gesamtgewicht - bevorzugt in Mengen von 0,001 bis 2 Gew.-%, mehr bevorzugt von 0,0025 bis 1,5 Gew.-%, und insbesondere von 0,005 bis 1 Gew.-% eingesetzt werden. Besonders bevorzugt enthält eine erfindungsgemäße Zusammensetzung - bezogen auf ihr Gesamtgewicht - 0,005 bis 1 Gew.-%, insbesondere 0,01 bis 1 Gew.-% Hyaluronsäure. Weiterhin besonders bevorzugt enthält eine erfindungsgemäße Zusammensetzung - bezogen auf ihr Gesamtgewicht-0,1 bis 5 Gew.-%, insbesondere 0,5 bis 3 Gew.-% Hyaluronic Filling Spheres^{®}.

Komponente (e) kann den erfindungsgemäßen Zusammensetzungen sowohl separat zugesetzt werden, als auch Bestandteil der zuvor beschriebenen kosmetischen und/oder dermatologischen Trägers sein.

Bevorzugte Komponenten (e) sind beispielsweise 1,2-Propylenglycol, Glycerin, Diglycerin, Triglycerin, 1,2-Butandiol, 1,3-Butandiol, 1,2-Pentandiol, 1,5-Pentandiol, 1,2-Hexandiol, 1,6-Hexandiol, Sorbitol und/oder Sorbitan.

Besonders bevorzugt sind 1,2-Propylenglycol, Glycerin, und/oder 1,6-Hexandiol sowie Mischungen dieser Alkohole. Insbesondere bevorzugt sind Glycerin und/oder 1,6-Hexandiol.

Die Komponente(n) (e) können in den erfindungsgemäßen Zusammensetzungen - bezogen auf deren Gesamtgewicht - bevorzugt in Mengen von 1 bis 90 Gew.-%, mehr bevorzugt von 1 bis 70 Gew.-%, besonders bevorzugt von 1 bis 50 Gew.-% und insbesondere 1 bis 30 Gew.-% eingesetzt werden.

Besonders bevorzugt enthält eine erfindungsgemäße Zusammensetzung - bezogen auf ihr Gesamtgewicht - 1 bis 30 Gew.-%, insbesondere 1 bis 20 Gew.-% Glycerin und/oder 1,6-Hexandiol.

In einer ersten bevorzugten Ausführungsform der Erfindung enthält eine erfindungsgemäße Zusammensetzung - bezogen auf ihr Gesamtgewicht -
- 0,001 bis 2 Gew.-% mindestens einer der zuvor beschriebenen Komponente(n) (a),
- 0,01 bis 3 Gew.-% mindestens einer der zuvor beschriebenen Komponente(n) (b),
- 0,01 bis 5 Gew.-% mindestens einer der zuvor beschriebenen Komponente(n) (c),
- 0,001 bis 2 Gew.-% mindestens einer der zuvor beschriebenen Komponente(n) (d) und
- 1 bis 90 Gew.-% mindestens einer der zuvor beschriebenen Komponente(n) (e).

Innerhalb dieser Ausführungsform ist es besonders bevorzugt, wenn die erfindungsgemäße Zusammensetzung, bezogen auf ihr Gesamtgewicht,
- 0,001 bis 2 Gew.-%, mehr bevorzugt 0,0025 bis 1,5 Gew.-% und insbesondere 0,005 bis 1 Gew.-% mindestens einer der unter der INCI-Bezeichnungen Bifonazole, Climbazole, Clotrimazole, Econazole, Ketoconazole, Miconazole bekannten Verbindungen,
- 0,01 - 3 Gew.-%, mehr bevorzugt 0,05 bis 2 Gew.-% und insbesondere 0,1 bis 1 Gew.-% alpha-Tocopherol und/oder mindestens einen Ester des alpha-Tocopherols wie Tocopherylacetat, -nicotinat, -phosphat, -succinat, -linoleat, -oleat, Tocophereth-5, Tocophereth-10, Tocophereth-12, Tocophereth-18, Tocophereth-50 und/oder Tocophersolan,
- 0,01 - 5 Gew.-%, mehr bevorzugt 0,025 bis 3 Gew.-% und insbesondere 0,05 bis 1 Gew.-% Glycyrrhitinsäure und/oder ein physiologisch verträgliches Salz der Glycyrrhitinsäure,
- 0,001 bis 2 Gew.-%, mehr bevorzugt 0,0025 bis 1,5 Gew.-%, und insbesondere 0,005 bis 1 Gew.-% Hyaluronsäure und/oder ein physiologisch verträgliches Salz der Hyaluronsäure und
- 1 bis 90 Gew.-%, mehr bevorzugt von 1 bis 70 Gew.-%, besonders bevorzugt von 1 bis 50 Gew.-% und insbesondere 1 bis 30 Gew.-% 1,2-Propylenglycol, Glycerin, Diglycerin, Triglycerin, 1,2-Butandiol, 1,3-Butandiol, 1,2-Pentandiol, 1,5-Pentandiol, 1,2-Hexandiol, 1,6-Hexandiol, Sorbitol und/oder Sorbitan enthält.

Insbesondere bevorzugte erfindungsgemäße Zusammensetzungen dieser Ausführungsform enthalten - bezogen auf ihr Gesamtgewicht -
- 0,005 bis 1 Gew.-% und insbesondere 0,01 bis 0,5 Gew.-% Climbazole,
- 0,05 bis 2 Gew.-%, insbesondere 0,1 bis 1 Gew.-% alpha-Tocopherol und/oder alpha-Tocopherolacetat,
- 0,05 bis 1 Gew.-%, insbesondere 0,1 bis 1 Gew.-% Glycyrrhitinsäure und/oder Ammonium Glycyrrhizate,
- 0,005 bis 1 Gew.-%, insbesondere 0,01 bis 1 Gew.-% Hyaluronsäure und/oder 0,1 bis 5 Gew.-%, insbesondere 0,5 bis 3 Gew.-% Hyaluronic Filling Spheres^{®}, und
- 1 bis 30 Gew.-%, insbesondere 1 bis 20 Gew.-% Glycerin und/oder 1,6-Hexandiol.

Wünschenswert und ein weiteres Ziel der Erfindung ist, dass die erfindungsgemäßen Zusammensetzungen neben der Antifaltenwirkung und/oder der Erhöhung der Hautfeuchtigkeit verbesserte Pflegeeffekte auf der Haut und/oder den Hautanhangsgebilden zeigen.

Verbesserte Pflegeeffekte sind beispielsweise durch das Auftreten eines verfeinerten Hautbildes (makroskopisch), eines frischeren Aussehens der Haut und/oder einer elastischen, weichen Hautoberfläche erkennbar.

In einer weiteren bevorzugten Ausführungsform enthalten erfindungsgemäße Zusammensetzungen zur Erzielung verbesserter Pflegeeffekte auf der Haut zusätzlich zu den zuvor beschriebenen Wirkstoffen (a) bis (e) bevorzugt mindestens einen Wirkstoff, ausgewählt aus
- Harnstoff-Derivaten, ausgewählt aus Verbindungen gemäß Formel (I) 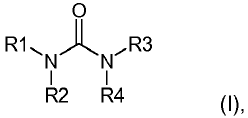 worin
   die Reste R1, R2, R3 und R4 unabhängig voneinander für ein Wasserstoffatom, eine C₁-C₄-Alkylgruppe, eine C₂-C₆-Alkenylgruppe oder eine C₂-C₆-Hydroxyalkylgruppe mit mindestens einer Hydroxygruppe steht,
   mit der Maßgabe, dass mindestens einer der besagten Reste eine C₂-C₆Hydroxyalkylgruppe mit mindestens einer Hydroxygruppe bedeutet,
- Vitaminen, Provitaminen und Vitaminvorstufen der Gruppen B, C, H und K und den Estern der vorgenannten Substanzen,
- Monomeren, Oligomeren und Polymeren von Aminosäuren, N-C₂-C₂₄-Acylaminosäuren, den Estern und/oder den physiologisch verträglichen Metallsalzen dieser Substanzen,
- DNA- oder RNA-Oligonucleotiden,
- natürlichen Betainverbindungen,
- α-Hydroxycarbonsäuren, α-Ketocarbonsäuren, β-Hydroxycarbonsäuren und deren Ester-, Lacton- oder Salzform, ausgenommen Zusammensetzungen mit 5 Gew.-% (2-Hydroxyethyl)harnstoff und 0,05 bzw. 5 Gew.-% Ammoniumlactat und Zusammensetzungen mit (2-Hydroxypropyl)harnstoff und α- und/oder β-Hydroxycarbonsäuren,
- den Flavonoiden Naringin, α-Glucosylrutin, α-Glucosylmyricetin, α-Glucosylisoquercetin, α-Glucosylquercetin, Dihydroquercetin (Taxifolin), Hesperidin, Hesperitin, Neohesperidin, Rutin, Troxerutin, Monoxerutin, Diosmin, Eriodictin und Apigenin-7-glucosid,
- Isoflavonoiden und Isoflavonoid-reichen Pflanzenextrakten,
- Ubichinon und Ubichinol sowie deren Derivaten,
- Silymarin,
- natürlich vorkommenden Xanthin-Derivaten, ausgewählt aus Coffein, Theophyllin, Theobromin und Aminophyllin,
- Ectoin,
- Kreatin,
- Olivenblattextrakten, Ursolsäure, Oleanol und/oder Oleanolsäure,
- Mono- und Polyhydroxystilbenen und deren Estern,
- Derivaten von methyliertem Silanol,
- Phytinsäure,
- Extrakten aus Maiskörnern (Zea Mays (Corn) Kernel Extract),
- Extrakten aus Haferkörnern (Avena Sativa (Oat) Kernel Extract),
- Saccharomyces/Xylinum/Black Tea Ferment,
- Apfelkernextrakten (Pyrus Malus (Apple) Fruit Extract),
- Lotuskeim-Extrakten (Nelumbo Nucifera Germ Extract),
- Rotweinextrakten,
- Traubenkernextrakten (Vitis Vinifera (Grape) Seed Extract), die bevorzugt aus der Chardonnay-Traube stammen,
- Extrakten aus Schwarzen Holunderblüten (Sambucus Nigra Flower Extract),
- Wirkstoffen, die die beta-Endorphinsynthese in Keratinozyten stimulieren,
- anorganischen und organischen UV-Filtersubstanzen,
- selbstbräunenden Wirkstoffen,
- hautaufhellenden Wirkstoffen,
- Wirkstoffen, die die Prostaglandinsynthese und/oder die Leukotrien-Synthese inhibieren,
- sebumregulierenden Wirkstoffen,
- sowie Desoxyzuckern oder Desoxyzucker-Bausteine enthaltenden Polysacchariden.

Besonders bevorzugte Harnstoffderivate der Formel (I) sind solche, die mindestens eine C₂-C₆-Hydroxyalkylgruppe mit mindestens einer Hydroxygruppe enthalten, welche aus mindestens einem Vertreter aus der Gruppe aus 2-Hydroxyethyl, 2-Hydroxy-2-methylprop-2-yl, 1,3-Dihydroxy-2-methylprop-2-yl, 1,3-Dihydroxyprop-2-yl, Tris(hydroxymethyl)methyl, 1,3-Dihydroxy-2-hydroxymethylprop-2-yl, 2,3-Dihydroxypropyl, 2-Hydroxypropyl, 3-Hydroxypropyl, 4-Hydroxy-butyl, 1-Hydroxy-2-methylprop-2-yl, 2,3,4,5,6-Pentahydroxyhexyl und 1,3,4,5,6-Pentahydroxyhex-2-yl ausgewählt wird.

Es ist bevorzugt solche Harnstoffderivate der Formel (I) zu verwenden, welche die bevorzugte Maßgabe der Formel (I) erfüllen, dass mindestens einer der Reste R1 bis R4 eine C₂-C₆-Hydroxyalkylgruppe mit mindestens einer Hydroxygruppe bedeutet. Weiterhin besonders bevorzugt stehen jeweils zwei Reste aus R1, R2, R3 und R4 für eine C₂-C₆-Hydroxyalkylgruppe mit mindestens einer Hydroxygruppe. Insbesondere bevorzugt stehen die zwei Reste aus R1, R2, R3 und R4 für eine C₂-C₆-Hydroxyalkylgruppe mit mindestens einer Hydroxygruppe, wobei diese beiden Reste an unterschiedlichen Stickstoffatomen des Harnstoffs positioniert (N,N'-Stellung) sind. Beispiele für in Verbindungen der Formel (I) verwendbare C₁-C₄-Alkylgruppen sind Methyl, Ethyl, n-Propyl, Isopropyl, n-Butyl, sec-Butyl, Isobutyl und tert-Butyl. Beispiele für in Verbindungen der Formel (I) verwendbare C₂-C₆-Alkenylgruppen sind Vinyl, 2-Propen-1-yl (Allyl) oder 3-Buten-1-yl.

Besonders bevorzugt wird mindestens eine Verbindung aus der Gruppe mit den Vertretern N-(2-Hydroxyethyl)harnstoff, N,N-Bis-(2-hydroxyethyl)harnstoff, N,N'-Bis-(2-hydroxyethyl)harnstoff, N-(3-Hydroxypropyl)harnstoff, N,N-Bis-(3-hydroxypropyl)harnstoff, N,N'-Bis-(3-hydroxypropyl)harnstoff, N-(2-Hydroxypropyl)harnstoff, N,N-Bis-(2-hydroxypropyl)harnstoff, N,N'-Bis-(2-hydroxy-propyl)harnstoff, N-(2-Hydroxy-2-methyl-prop-2-yl)harnstoff, N,N-Bis-(2-hydroxy-2-methyl-prop-2-yl)harnstoff, N,N'-Bis-(2-hydroxy-2-methyl-prop-2-yl)harnstoff, N-(1,3-Dihydroxy-2-methyl-prop-2-yl)harnstoff, N,N-Bis-(1,3-dihydroxy-2-methyl-prop-2-yl)harnstoff, N,N'-Bis-(1,3-dihydroxy-2-methyl-prop-2-yl)harnstoff, N-(1,3-Dihydroxy-2-hydroxymethyl-prop-2-yl)harnstoff, N,N-Bis-(1,3-dihydroxy-2-hydroxymethyl-prop-2-yl)harnstoff und N,N'-Bis-(1,3-dihydroxy-2-hydroxymethyl-prop-2-yl)harnstoff ausgewählt. Ganz besonders bevorzugt ist N-(2-Hydroxyethyl)harnstoff und N,N'-Bis-(2-hydroxyethyl)harnstoff, erhältlich beispielsweise als Handelsprodukt Hydrovance^{®} von der Firma National Starch.

Erfindungsgemäße Zusammensetzungen enthalten Harnstoff-Derivate der zuvor genannten Formel (I) bevorzugt in Mengen von 0,75 bis 4,5 Gew.-%, mehr bevorzugt von 1 bis 4 Gew.-%, besonders bevorzugt von 2 bis 3,5 Gew.-% und insbesondere von 2,5 bis 3,2 Gew.-%, wobei sich die Mengenangaben auf das Gesamtgewicht der Zusammensetzung beziehen.

Geeignete Vitamine, Provitamine oder Vitaminvorstufen der Vitamingruppen B, C, H und K können beispielsweise ausgewählt sein aus:
i) Vitamin B: Zur Vitamin B-Gruppe oder zu dem Vitamin B-Komplex gehören unter anderem
   - Vitamin B₁: Trivialname Thiamin, chemische Bezeichnung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in den erfindungsgemäßen Zusammensetzungen in Mengen von 0,05 bis 1 Gew.-%, bezogen auf die gesamte Zusammensetzung, eingesetzt,
   - Vitamin B₂: Trivialname Riboflavin, chemische Bezeichnung 7,8-Dimethyl-10-(1-D-ribityl)-benzo[g]pteridin-2,4(3H,10H)-dion. Bevorzugt werden Riboflavin oder seine Derivate in den erfindungsgemäßen Zusammensetzungen in Mengen von 0,05 bis 1 Gew.-%, bezogen auf die gesamte Zusammensetzung, eingesetzt,
   - Vitamin B₃: Unter dieser Bezeichnung werden die Verbindungen Nicotinsäure und Nicotinsäureamid (Niacinamid) geführt. Bevorzugt ist Nicotinsäureamid, das in den erfindungsgemäßen Zusammensetzungen bevorzugt in Mengen von 0,05 bis 1 Gew.-%, bezogen auf die gesamte Zusammensetzung, enthalten sein kann,
   - Vitamin B₅: Pantothensäure und Panthenol. Einsetzbare Derivate des Panthenols sind insbesondere die Ester und Ether des Panthenols sowie kationisch derivatisierte Panthenole. An Stelle von sowie zusätzlich zu Pantothensäure oder Panthenol können die erfindungsgemäßen Zusammensetzungen auch Derivate des 2-Furanon mit der allgemeinen Strukturformel (II) enthalten, 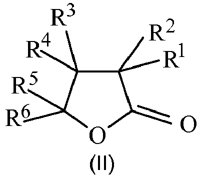 wobei 2-Furanon-Derivate bevorzugt sind, in denen die Substituenten R¹ bis R⁶ unabhängig voneinander ein Wasserstoffatom, einen Hydroxylrest, einen Methyl-, Methoxy-, Aminomethyl- oder Hydroxymethylrest, einen gesättigten oder ein- oder zweifach ungesättigten, linearen oder verzweigten C₂-C₄-Kohlenwasserstoffrest, einen gesättigten oder ein- oder zweifach ungesättigten, verzweigten oder linearen Mono-, Di- oder Trihydroxy-C₂-C₄ - Kohlenwasser stoffrest oder einen gesättigten oder ein- oder zweifach ungesättigten, verzweig ten oder linearen Mono-, Di- oder Triamino-C₂-C₄ - Kohlenwasserstoffrest dar stellen.
      Besonders bevorzugte Derivate sind die auch im Handel erhältlichen Substanzen Dihydro-3-hydroxy-4,4-dimethyl-2(3H)-furanon mit dem Trivialnamen Pantolacton (Merck), 4-Hydroxymethyl-γ-butyrolacton (Merck), 3,3-Dimethyl-2-hydroxy-γ-butyrolacton (Aldrich) und 2,5-Dihydro-5-methoxy-2-furanon (Merck), wobei ausdrücklich alle Stereoisomeren eingeschlossen sind. Ein außerordentlich bevorzugtes 2-Furanon-Derivat ist Pantolacton (Dihydro-3-hydroxy-4,4-dimethyl-2(3H)-furanon), wobei in Formel (II) R¹ für eine Hydroxylgruppe, R² für ein Wasserstoffatom, R³ und R⁴ für eine Methylgruppe und R⁵ und R⁶ für ein Wasserstoffatom stehen. Das Stereoisomer (R)-Pantolacton entsteht beim Abbau von Pantothensäure.
      Die genannten Verbindungen des Vitamin B₅-Typs sowie die 2-Furanonderivate können in den erfindungsgemäßen Zusammensetzungen in einer Gesamtmenge von 0,05 bis 5 Gew.-%, bevorzugt von 0,1 bis 3 Gew.-%, besonders bevorzugt von 0,5 bis 2 Gew.-%, jeweils bezogen auf die gesamte Zusammensetzung, enthalten sein,
   - Vitamin B₆: hierunter wird keine einheitliche Substanz, sondern die unter den Trivialnamen Pyridoxin, Pyridoxamin und Pyridoxal bekannten Derivate des 5-Hydroxymethyl-2-methylpyridin-3-ols verstanden. Vitamin B₆ kann in den erfindungsgemäßen Zusammensetzungen bevorzugt in Mengen von 0,0001 bis 1,0 Gew.-%, insbesondere in Mengen von 0,001 bis 0,01 Gew.-%, enthalten sein, wobei sich die Mengenangaben auf das Gesamtgewicht der Zusammensetzung beziehen,
   - Vitamin B₇: (Biotin), auch als Vitamin H oder "Hautvitamin" bezeichnet. Bei Biotin handelt es sich um (3a*S*,4*S*, 6a*R*)-2-Oxohexahydrothienol[3,4-*d*]-imidazol-4-valeriansäure. Biotin kann in den erfindungsgemäßen Zusammensetzungen bevorzugt in Mengen von 0,0001 bis 1,0 Gew.-%, insbesondere in Mengen von 0,001 bis 0,01 Gew.-% enthalten sein, wobei sich die Mengenangaben auf das Gesamtgewicht der Zusammensetzung beziehen,
(ii) Vitamin C (Ascorbinsäure): kann in den erfindungsgemäßen Zusammensetzungen bevorzugt in Mengen von 0,1 bis 3 Gew.-%, bezogen auf die gesamte Zusammensetzung, eingesetzt werden. Die Verwendung der Derivate Ascorbylpalmitat, -stearat, -dipalmitat, -acetat, Mg-Ascorbylphosphat, Na-Ascorbylphosphat, Natrium- und Magnesiumascorbat, Dinatriumascorbylphosphat und -sulfat, Kaliumascorbyltocopherylphosphat, Chitosanascorbat oder Ascorbylglucosid kann bevorzugt sein. Die Verwendung in Kombination mit Tocopherolen kann ebenfalls bevorzugt sein,
(iii) Vitamin F: Unter Vitamin F werden üblicherweise essentielle Fettsäuren, insbesondere Linolsäure, Linolensäure und Arachidonsäure, verstanden,
(iv) Vitamin H: Vitamin H ist eine andere Bezeichnung für Biotin oder Vitamin B₇ (siehe oben),
(v) Vitamin K: Zu den fettlöslichen Vitaminen der Vitamin K-Gruppe, denen das Grundgerüst des 2-Methyl-1,4-naphthochinons zugrunde liegt, gehören Phyllochinon (Vitamin K₁), Farnochinon oder Menachinon-7 (Vitamin K2) und Menadion (Vitamin K₃). Vitamin K kann in den erfindungsgemäßen Zusammensetzungen bevorzugt in Mengen von 0,0001 bis 1,0 Gew.-%, insbesondere 0,01 bis 0,5 Gew.-%, jeweils bezogen auf die gesamte Zusammensetzung, enthalten sein.

Panthenol, Pantolacton, Nicotinsäureamid, Pyridoxin, Pyridoxamin, Pyridoxal, Biotin, Ascorbylpalmitat, -acetat, Mg-Ascorbylphosphat, Na-Ascorbylphosphat und/oder Natrium- und Magnesiumascorbat besonders Panthenol und Pantolacton sind besonders bevorzugt.

Geeignete Monomere von Aminosäuren und/oder der N-C₂-C₂₄-Acylaminosäuren können ausgewählt sein aus Alanin, Arginin, Asparagin, Asparaginsäure, Canavanin, Citrullin, Cystein, Cystin, Desmosin, Glutamin, Glutaminsäure, Glycin, Histidin, Hydroxylysin, Hydroxyprolin, Isodesmosin, Isoleucin, Leucin, Lysin, Methionin, Ornithin, Phenylalanin, Prolin, Pyroglutaminsäure, Sarcosin, Serin, Taurin, Threonin, Thyroxin, Tryptophan, Tyrosin und Valin. Besonders bevorzugt sind Taurin, Arginin, Hydroxyprolin, Prolin und Glutaminsäure.

Der C₂ - C₂₄-Acylrest, mit dem die genannten Aminosäuren an der Aminogruppe derivatisiert sind, ist bevorzugt ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten. Bevorzugte Beispiele sind Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Besonders bevorzugt sind Zinkpyroglutamat und Natriumlauroylglutamat.

Physiologisch verträgliche Salze der zuvor genannten Wirkstoffe, die Säuregruppen enthalten und Salze bilden können, können ausgewählt sein aus den Ammonium-, Alkalimetall-, Magnesium-, Calcium-, Aluminium-, Zink- und Mangan-Salzen. Bevorzugt sind die Natrium-, Kalium-, Magnesium-, Aluminium-, Zink- und Mangan-Salze.

Geeignete Oligomere von Aminosäuren und/oder der N-C₂-C₂₄-Acylaminosäuren können ausgewählt sein aus Di-, Tri-, Tetra-, Penta-, Hexa-, Hepta-, Octa-, Nona- und Decapeptiden, die acyliert und/oder verestert sein können. Besonders bevorzugt sind die Di-, Tri-, Tetra-, Penta- und Hexapeptide, die acyliert und/oder verestert sein können. Bevorzugte, gegebenenfalls acylierte und/oder veresterte Dipeptide sind Tyr-Arg, Val-Trp, Asn-Phe, Asp-Phe, N-Palmitoyl-ß-Ala-His, N-Acetyl-Tyr-Arg-hexyldecylester (z.B. Calmosensine von Sederma), Carnosin (ß-Ala-His) und N-Palmitoyl-Pro-Arg. Bevorzugte, gegebenenfalls acylierte und/oder veresterte Tripeptide sind Gly-His-Lys, N-Palmitoyl-Gly-His-Lys, Gly-Lys-His, His-Ala-Orn, Lys-Phe-Lys, N-Elaidoyl-Lys-Phe-Lys und N-Acetyl-Arg-Lys-Arg-NH₂. Bevorzugte, gegebenenfalls acylierte und/oder veresterte Tetrapeptide sind Gly-Gln-Pro-Arg, Gly-Gln-Arg-Pro und N-Palmitoyl-Gly-Gln-Pro-Arg. Bevorzugte, gegebenenfalls acylierte und/oder veresterte Pentapeptide sind Lys-Thr-Thr-Lys-Ser, N-Palmitoyl-Lys-Thr-Thr-Lys-Ser, N-Palmitoyl-Tyr-Gly-Gly-Phe-Met und N-Palmitoyl-Tyr-Gly-Gly-Phe-Leu. Ein bevorzugtes Hexapeptid ist Palmitoyl-Val-Gly-Val-Ala-Pro-Gly (Biopeptide EL von Sederma).

Es kann besonders bevorzugt sein, dass die erfindungsgemäßen Zusammensetzungen ein Gemisch aus mindestens zwei Oligopeptiden enthalten. Ein besonders bevorzugtes Gemisch ist die Kombination aus N-Palmitoyl-Gly-His-Lys (z.B. Biopeptide CL von Sederma) und N-Palmitoyl-Gly-Gln-Pro-Arg (z.B. in Eyeliss von Sederma). Eine vorgefertigte Mischung des Tripeptids Palmitoyl-Gly-His-Lys und des Tetrapeptids N-Palmitoyl-Gly-Gln-Pro-Arg ist unter dem Handelsnamen Matrixyl 3000, ebenfalls von Sederma, erhältlich.

Geeignete Polymere von Aminosäuren und/oder der N-C₂-C₂₄-Acylaminosäuren können ausgewählt sein aus pflanzlichen und tierischen Proteinhydrolysaten und/oder Proteinen. Unter tierischen Proteinhydrolysaten sind z. B. Elastin-, Collagen-, Keratin-, Seiden- und Milcheiweiß-Proteinhydrolysate zu verstehen, die auch in Form von Salzen vorliegen können. Bevorzugt sind pflanzliche Proteinhydrolysate, z. B. Soja-, Weizen-, Mandel-, Erbsen-, Kartoffel- und Reisproteinhydrolysate. Entsprechende Handelsprodukte sind z. B. DiaMin^{®} (Diamalt), Gluadin^{®} (Cognis), Lexein^{®} (Inolex) und Crotein^{®} (Croda). Besonders bevorzugt sind Sojaproteinhydrolysate, z. B. die Handelsprodukte Phytokine^{®} von Coletica oder Ridulisse C^{®} von Silab.

Proteinhydrolysate können naturgemäß auch monomere Aminosäuren und Oligopeptide enthalten; ihre Zusammensetzung ist normalerweise nicht definiert.

Ebenfalls möglich ist der Einsatz von Acylderivaten der Proteinhydrolysate, z. B. in Form ihrer Fettsäure-Kondensationsprodukte. Entsprechende Handelsprodukte sind z. B. Lamepon^{®} (Cognis), Gluadin^{®} (Cognis), Lexein^{®} (Inolex), Crolastin^{®} oder Crotein^{®} (Croda). Erfindungsgemäß einsetzbar sind auch kationisierte Proteinhydrolysate.

In einer weiteren bevorzugten Ausführungsform können die Polymeren der Aminosäuren ausgewählt seinaus DNA-Reparaturenzymen.

Bevorzugte DNA-Reparaturenzyme sind Photolyase und T4 Endonuclease V, letztere im Weiteren mit "T4N5" abgekürzt. Diese beiden Enzyme sind im Stand der Technik bereits als so genannte DNA-Reparatur-Enzyme bekannt. Unter DNA-Reparatur ist definitionsgemäß die Spaltung bzw. Entfernung von UV-induzierten Pyrimidindimeren aus der DNA zu verstehen.

Besonders bevorzugt ist der Einsatz von liposomenverkapselten DNA-Reparaturenzymen. Liposomenverkapselte Photolyase ist im Handel z. B. unter der Produktbezeichnung Photosome™, liposomenverkapselte T4N5 z. B. unter der Bezeichnung Ultrasome™ von der Firma AGI Dermatics, USA, erhältlich.

In den erfindungsgemäßen Zusammensetzungen können die Photosome™ oder Ultrasome™ bevorzugt in Mengen von 0,1 - 10 Gew.-%, mehr bevorzugt von 0,5 - 5,0 Gew.-% und besonders bevorzugt von 1,0 - 4,0 Gew.-%, bezogen auf die gesamte Zusammensetzung, enthalten sein.

In den erfindungsgemäßen Zusammensetzungen können die Monomere, Oligomere oder Polymere von Aminosäuren, N-C₂-C₂₄-Acylaminosäuren und/oder den Estern und/oder den physiologisch verträglichen Metallsalzen dieser Substanzen bevorzugt in Mengen von 0,0001 - 10 Gew.-%, mehr bevorzugt von 0,01 - 5 Gew.-% und besonders bevorzugt von 0,1 - 3 Gew.-%, jeweils bezogen auf die gesamte Zusammensetzung, enthalten sein.

Unter geeigneten DNA- oder RNA-Oligonucleotiden werden bevorzugt Polymerisate aus 2 bis 20, mehr bevorzugt aus 2 bis 10 Mononucleotiden verstanden, die ebenso wie bei Polynucleotiden und Nucleinsäuren durch Phosphorsäurediester-Brücken verknüpft sind. Die Nucleotide bestehen aus Nucleobasen (meist Pyrimidin- oder Purin-Derivaten), Pentosen (meist D-Ribofuranose oder 2-Desoxy-D-ribofuranose in β-N-glykosidischer Bindung an die Nucleobase) und Phosphorsäure. Die Mononucleotide sind zum Beispiel Adenosinphosphate, Cytidinphosphate, Guanosinphosphate, Uridinphosphate und Thymidinphosphate, insbesondere CMP (Cytidin-5'-monophosphat), UDP (Uridin-5'-diphosphat), ATP (Adenosin-5 '-triphosphat) und GTP (Guanosin-5'-triphosphat).

Ein besonders bevorzugtes Oligonucleotid ist das Thymidin-Dinucleotid.

In den erfindungsgemäßen Zusammensetzungen können die DNA-Oligonucleotide oder RNA-Oligonucleotide vorzugsweise in Mengen von 0,0001 - 5 Gew.-%, bevorzugt von 0,001 - 1,0 Gew.-% und besonders bevorzugt von 0,01 - 0,5 Gew.-%, bezogen auf die gesamte Zusammensetzung, enthalten sein.

Geeignete natürliche Betainverbindungen sind bevorzugt natürlich vorkommende Verbindungen mit der Atomgruppierung R₃N⁺-CH₂-X-COO⁻ gemäß IUPAC-Regel C-816.1. So genannte Betaintenside (synthetisch) fallen nicht unter die geeigneten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Bevorzugte Betainverbindungen sind Betain (Me₃N⁺-CH₂-COO⁻) und Carnitin (Me₃N⁺-CH₂-CHOH-CH₂-COO⁻), jeweils mit Me = Methyl und X = C-C-Einfachbindung (im Falle des Betains) oder X = -CHOH-CH₂- (im Falle des Carnitins).

Die Betainverbindungen können in den erfindungsgemäßen Zusammensetzungen vorzugsweise in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten sein.

Unter geeigneten α-Hydroxycarbonsäuren, α-Ketocarbonsäuren oder β-Hydroxycarbonsäuren oder deren Ester-, Lacton- oder Salzformen werden bevorzugt Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure verstanden. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Die Ester der genannten Säuren können ausgewählt sein aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Die α-Hydroxycarbonsäuren, α-Ketocarbonsäuren oder β-Hydroxycarbonsäuren oder ihre Derivate können in den erfindungsgemäßen Zusammensetzungen verwendeten Mitteln vorzugsweise in Mengen von 0,1 - 10 Gew.-%, bevorzugt von 0,5 - 5 Gew.-% enthalten sein, wobei sich die Mengenangaben auf die gesamte Zusammensetzung beziehen.

Bevorzugte Flavonoide umfassen die Glycoside der Flavone, der Flavanone, der 3-Hydroxyflavone (Flavonole), der Aurone und der Isoflavone. Besonders bevorzugte Flavonoide können ausgewählt sein aus Naringin (Aurantiin, Naringenin-7-rhamnoglucosid), α-Glucosylrutin, α-Glucosylmyricetin, α-Glucosylisoquercetin, α-Glucosylquercetin, Hesperidin (3',5,7-Trihydroxy-4'-methoxyflavanon-7-rhamnoglucosid, Hesperitin-7-O-rhamnoglucosid), Neohesperidin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxyethoxy)-flavon-3-(6-O-(6-deoxy-α-L-mannopyranosyl)-β-D-glucopyranosid)), Diosmin (3',4',7-Trihydroxy-5-methoxyflavanon-7-rhamnoglucosid), Eriodictin und Apigenin-7-glucosid (4',5,7-Trihydroxyflavon-7-glucosid).

Außerordentlich bevorzugte Flavonoide sind α-Glucosylrutin, Naringin und Apigenin-7-glucosid. Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin, Hesperidinmethylchalkon und Neohesperidindihydrochalkon.

Die Flavonoide können in den erfindungsgemäßen Zusammensetzungen vorzugsweise in Mengen von 0,0001 bis 1 Gew.-%, bevorzugt von 0,0005 bis 0,5 Gew.-% und besonders bevorzugt von 0,001 bis 0,1 Gew.-% enthalten sein, jeweils bezogen auf die Flavonoidaktivsubstanz in der gesamten kosmetischen Zusammensetzung.

Zu den geeigneten Isoflavonoiden werden bevorzugt die Isoflavone und die Isoflavon-Glycoside gezählt.

Unter Isoflavonen sind im Sinne der vorliegenden Erfindung Stoffe zu verstehen, die Hydrierungs-, Oxidations- oder Substitutionsprodukte des 3-Phenyl-4H-1-benzopyrans darstellen, wobei eine Hydrierung in der 2,3-Stellung des Kohlenstoffgerüsts vorliegen kann, eine Oxidation unter Ausbildung einer Carbonylgruppe in der 4-Stellung vorliegen kann, und unter Substitution der Ersatz eines oder mehrerer Wasserstoffatome durch Hydroxy- oder Methoxy-Gruppen zu verstehen ist. Zu den bevorzugten Isoflavonen zählen beispielsweise Daidzein, Genistein, Prunetin, Biochanin, Orobol, Santal, Pratensein, Irigenin, Glycitein, Biochanin A und Formononetin. Als Isoflavone besonders bevorzugt sind Daidzein, Genistein, Glycitein und Formononetin.

In bevorzugten Isoflavon-Glycosiden sind die Isoflavone über mindestens eine Hydroxygruppe mit mindestens einem Zucker glycosidisch verknüpft. Als Zucker kommen Mono- oder Oligosaccharide, insbesondere D-Glucose, D-Galactose, D-Glucuronsäure, D-Galacturonsäure, D-Xylose, D-Apiose, L-Rhamnose, L-Arabinose und Rutinose in Betracht.

Besonders bevorzugte Isoflavon-Glycoside sind Daidzin und Genistin.

Weiterhin bevorzugt ist es, wenn die Isoflavone und/oder deren Glycoside als Bestandteile eines aus einer Pflanze gewonnenen Substanzgemisches, insbesondere eines pflanzlichen Extraktes, in den Zubereitungen enthalten sind. Solche pflanzlichen Substanzgemische können in dem Fachmann geläufiger Weise beispielsweise durch Auspressen oder Extrahieren aus Pflanzen wie Soja, Rotklee oder Kichererbsen gewonnen werden. Besonders bevorzugt können Isoflavone oder Isoflavon-Glycoside in Form von aus Soja gewonnenen Extrakten in den erfindungsgemäß verwendeten Mitteln eingesetzt werden, wie sie beispielsweise unter der Produktbezeichnung Soy Protein Isolate SPI (Protein Technology International, St. Louis) oder Soy Phytochemicals Concentrate SPC (Archer Daniels Midland, Decatur) im Handel erhältlich sind. Ein weiterer besonders bevorzugter Isoflavonoid-reicher Pflanzenextrakt ist Apfelkernextrakt, insbesondere das Handelsprodukt Ederline von Seporga. Ederline enthält Phytohormone, Isoflavonoide, Phytosterole, Triterpenoide, Tocopherole und natürliche Wachse.

Die Isoflavonoide können in den erfindungsgemäßen Zusammensetzungen vorzugsweise in Mengen von 0,00001 bis 1 Gew.-%, bevorzugt von 0,0005 bis 0,5 Gew.-% und besonders bevorzugt von 0,001 bis 0,1 Gew.-% enthalten sein, jeweils bezogen auf die Isoflavonoidaktivsubstanz in der gesamten kosmetischen Zusammensetzung.

In einer weiteren bevorzugten Ausführungsform können die erfindungsgemäßen Zusammensetzungen zusätzlich mindestens ein Polyphenol oder einen Polyphenol-reichen Pflanzenextrakt enthalten.

Unter Polyphenolen sind bevorzugt aromatische Verbindungen zu verstehen, die mindestens zwei phenolische Hydroxy-Gruppen im Molekül enthalten. Hierzu zählen die drei Dihydroxybenzole Brenzcatechin, Resorcin und Hydrochinon, weiterhin Phloroglucin, Pyrogallol und Hexahydroxybenzol. In der Natur treten freie und veretherte Polyphenole beispielsweise in Blütenfarbstoffen (Anthocyanidine, Flavone), in Gerbstoffen (Catechine, Tannine), als Flechten- oder Farn-Inhaltsstoffe (Usninsäure, Acylpolyphenole), in Ligninen und als Gallussäure-Derivate auf. Bevorzugte Polyphenole sind Flavone, Catechine, Usninsäure, und als Tannine die Derivate der Gallussäure, Digallussäure und Digalloylgallussäure. Besonders bevorzugte Polyphenole sind die monomeren Catechine, das heißt die Derivate der Flavan-3-ole, und Leukoanthocyanidine, das heißt die Derivate der Leukoanthocyanidine, die bevorzugt in 5,7,3',4',5'-Stellung phenolische Hydroxygruppen tragen, bevorzugt Epicatechin und Epigallocatechin, sowie die daraus durch Selbstkondensation entstehenden Gerbstoffe. Solche Gerbstoffe werden bevorzugt nicht in isolierter Reinsubstanz, sondern als Extrakte gerbstoffreicher Pflanzenteile eingesetzt, z. B. Extrakte von Catechu, Quebracho, Eichenrinde und Pinienrinde sowie anderen Baumrinden, Blättern von Grünem Tee (camellia sinensis) und Mate. Ebenfalls besonders bevorzugt sind die Tannine.

Ein besonders bevorzugter Polyphenol-reicher kosmetischer Wirkstoff ist das Handelsprodukt Sepivinol R, ein Extrakt aus Rotwein, erhältlich von der Firma Seppic. Ein weiterer besonders bevorzugter Polyphenol-reicher kosmetischer Wirkstoff ist das Handelsprodukt Crodarom Chardonnay L, ein Extrakt aus den Kernen der Chardonnay-Traube, erhältlich von der Firma Croda.

Die Polyphenole können in den erfindungsgemäßen Zusammensetzungen vorzugsweise in Mengen von 0,001 bis 10 Gew.-%, bevorzugt von 0,005 bis 5 Gew.-% und besonders bevorzugt von 0,01 bis 3 Gew.-%, jeweils bezogen auf die gesamte kosmetische Zusammensetzung, enthalten sein.

In einer weiteren bevorzugten Ausführungsform können die erfindungsgemäßen Zusammensetzungen weiterhin mindestens ein Ubichinon oder ein Ubichinol oder deren Derivate enthalten. Ubichinole sind die reduzierte Form der Ubichinone. Bevorzugte Ubichinone weisen die Formel (III) auf: 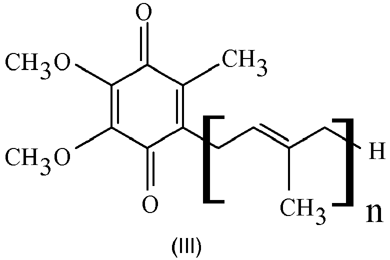 mit n = 6, 7, 8, 9 oder 10.

Besonders bevorzugt ist das Ubichinon der Formel (III) mit n = 10, auch bekannt als Coenzym Q10.

Die Ubichinone, Ubichinole oder deren Derivate können in den erfindungsgemäßen Zusammensetzungen vorzugsweise in Mengen von 0,0001 bis 1 Gew.-%, bevorzugt von 0,001 bis 0,5 Gew.-% und besonders bevorzugt von 0,005 bis 0,1 Gew.-%, jeweils bezogen auf die gesamte Zusammensetzung, enthalten sein.

Silymarin stellt ein früher als einheitliche Substanz angesehenes Wirkstoff-Konzentrat aus den Früchten der Mariendistel (Silybum marianum) dar. Die Hauptbestandteile des Silymarins sind Silybin (Silymarin I), Silychristin (Silymarin II) und Silydianin, die zur Gruppe der Flavanolignane gehören.

Silymarin kann in den erfindungsgemäßen Zusammensetzungen vorzugsweise in Mengen von 0,0001 bis 1 Gew.-%, bevorzugt von 0,001 bis 1,0 Gew.-% und besonders bevorzugt von 0,005 bis 0,5 Gew.-%, jeweils bezogen auf die gesamte Zusammensetzung, enthalten sein.

In einer weiteren bevorzugten Ausführungsform können die erfindungsgemäßen Zusammensetzungen mindestens ein natürlich vorkommendes Xanthin-Derivat, ausgewählt aus Coffein, Theophyllin, Theobromin und Aminophyllin, enthalten. Besonders bevorzugt ist Coffein.

Die natürlich vorkommenden Xanthin-Derivate können in den erfindungsgemäßen Zusammensetzungen vorzugsweise in Mengen von 0,0001 bis 5 Gew.-%, bevorzugt von 0,001 bis 3 Gew.-% und besonders bevorzugt von 0,005 bis 1 Gew.-%, jeweils bezogen auf die gesamte Zusammensetzung, enthalten sein.

Ectoin ist der Trivialname für 2-Methyl-1,4,5,6-tetrahydropyrimidin-4-carboxylat. Ectoin kann in den erfindungsgemäßen Zusammensetzungen vorzugsweise in Mengen von 0,0001 bis 1 Gew.-%, bevorzugt von 0,001 bis 0,5 Gew.-% und besonders bevorzugt von 0,005 bis 0,01 Gew.-%, jeweils bezogen auf die gesamte Zusammensetzung, enthalten sein.

Bei geeigneten UV-Filtersubstanzen handelt es sich bevorzugt um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme, wieder abzugeben. Man unterscheidet UVA-Filter und UVB-Filter. Die UVA- und UVB-Filter können sowohl einzeln als auch in Mischungen eingesetzt werden. Der Einsatz von Filter-Mischungen ist bevorzugt.

Besonders geeignete UV-Filter können ausgewählt sein aus den physiologisch verträglichen Derivaten von Dibenzoylmethan, Zimtsäureestern, Diphenylacrylsäureestern, Benzophenon, Campher, p-Aminobenzoesäureestern, o-Aminobenzoesäureestern, Salicylsäureestern, Benzimidazolen, symmetrisch oder unsymmetrisch substituierten 1,3,5-Triazinen, monomeren und oligomeren 4,4-Diarylbutadiencarbonsäureestern und -carbonsäureamiden, Ketotricyclo(5.2.1.0)decan, Benzalmalonsäureestern, Benzoxazol sowie beliebigen Mischungen der genannten Komponenten. Die organischen UV-Filter können öllöslich oder wasserlöslich sein. Besonders bevorzugte öllösliche UV-Filter sind 1-(4-tert.-Butylphenyl)-3-(4'-methoxyphenyl)propan-1,3-dion (Parsol^{®} 1789), 1-Phenyl-3-(4'-isopropylphenyl)-propan-1,3-dion, 3-(4'-Methylbenzyliden)-D,L-campher, 4-(Dimethylamino)-benzoesäure-2-ethylhexylester, 4-(Dimethylamino)benzoesäure-2-octylester, 4-(Dimethylamino)-benzoesäureamylester, 4-Methoxyzimtsäure-2-ethylhexylester, 4-Methoxyzimtsäurepropylester, 4-Methoxy-zimtsäureisopentylester, 2-Cyano-3,3-phenylzimtsäure-2-ethylhexylester (Octocrylene), Salicylsäure-2-ethylhexylester, Salicylsäure-4-isopropylbenzylester, Salicylsäurehomomenthylester (3,3,5-Trimethyl-cyclohexylsalicylat), 2-Hydroxy-4-methoxybenzophenon, 2-Hydroxy-4-methoxy-4'-methylbenzophenon, 2,2'-Dihydroxy-4-methoxybenzophenon, 4-Methoxybenzmalonsäuredi-2-ethylhexylester, 2,4,6-Trianilino-(p-carbo-2'-ethyl-1'-hexyloxy)-1,3,5-triazin (Octyl Triazone, Uvinul^{®} T 150), Dimethicodiethylbenzal malonate (CAS-Nr. 207574-74-1, Parsol^{®} SLX), Dioctyl Butamido Triazone (Uvasorb^{®} HEB), 2,4-bis-[5-1(dimethylpropyl)benzoxazol-2-yl-(4-phenyl)-imino]-6-(2-ethylhexyl)-imino-1,3,5-triazin (CAS Nr. 288254-16-0, Uvasorb^{®} K2A) und sowie beliebige Mischungen der genannten Komponenten. Bevorzugte wasserlösliche UV-Filter sind 2-Phenylbenzimidazol-5-sulfonsäure, Phenylen-1,4-bis-(2-benzimidazyl)-3,3'-5,5'-tetrasulfonsäure und deren Alkali-, Erdalkali-, Ammonium-, Alkylammonium-, Alkanolammonium- und Glucammoniumsalze, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze.

Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'methoxyphenyl)propan-1,3-dion (z. B. Parsol^{®} 1789) in verschiedenen UV-B-Filtern herstellen. Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexylsalicylat. In diesen Kombinationen liegt das Gewichtsverhältnis von UV-B-Filter zu 1-(4-tert.-Butylphenyl)-3-(4'methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0.

Bevorzugte anorganische Lichtschutzpigmente sind feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen, so genannte Nanopigmente. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex^{®} T2000 (Merck). Als hydrophobe Coatingmittel kommen dabei vor allem Silicone und dabei speziell Trialkoxyoctylsilane oder Simethicone in Frage. Besonders bevorzugt sind Titandioxid und Zinkoxid.

Die organischen UV-Filtersubstanzen können in den erfindungsgemäßen Zusammensetzungen bevorzugt in Mengen von 0,01 - 30 Gew.-%, mehr bevorzugt von 0,05 - 20 Gew.-%, besonders bevorzugt von 0,1 - 15 Gew.-% und außerordentlich bevorzugt von 0,25 - 10 Gew.-%, jeweils bezogen auf die gesamte Zusammensetzung, enthalten sein.

Die anorganischen UV-Filtersubstanzen können in den erfindungsgemäßen Zusammensetzungen vorzugsweise in Mengen von 0,01 - 15 Gew.-%, bevorzugt von 0,05 - 10 Gew.-%, besonders bevorzugt von 0,1 - 5 Gew.-% und außerordentlich bevorzugt von 0,25 - 4,0 Gew.-%, jeweils bezogen auf die gesamte Zusammensetzung, enthalten sein.

Geeignete hautaufhellende Wirkstoffe können ausgewählt sein aus Ascorbinsäure, den Estern der Ascorbinsäure mit Phosphorsäure und/oder organischen C₂-C₂₀-Carbonsäuren sowie deren Alkali- und Erdalkalimetallsalzen, Kojisäure, Hydrochinon, Arbutin, Maulbeerbaumextrakt und Süßholzextrakt sowie Mischungen hiervon. Sowohl als Einzelsubstanz wie auch in Mischung bevorzugt sind die Ascorbinsäurederivate sowie Kojisäure bevorzugt. Besonders bevorzugt sind Natriumascorbylphosphat, Magnesiumascorbylphosphat, Ascorbylmonopalmitat, Ascorbyldipalmitat, Ascorbylmonostearat, Ascorbyldistearat, Ascorbylmonoethylhexanoat, Ascorbyldiethylhexanoat, Ascorbylmonooctanoat, Ascorbyldioctanoat, Ascorbylmonoisostearat und Ascorbyldiisostearat. Die erfindungsgemäß außerordentlich bevorzugten Ascorbinsäurederivate sind Natriumascorbylphosphat und Magnesiumascorbylphosphat.

Die hautaufhellenden Wirkstoffe können in den erfindungsgemäßen Zusammensetzungen vorzugsweise in einer Menge von 0,05 bis 5 Gew.-%, bevorzugt von 0,1 - 2 Gew-%, jeweils bezogen auf die gesamte Zusammensetzung, enthalten sein.

Geeignete hautberuhigende Wirkstoffe sind bevorzugt ausgewählt aus Farnesol, Allantoin, α-Bisabolol und α-Liponsäure.

Die hautberuhigenden Wirkstoffe können in den erfindungsgemäßen Zusammensetzungen vorzugsweise in Mengen von 0,001 bis 5 Gew.-%, bevorzugt von 0,005 bis 1 Gew.-%, jeweils bezogen auf die gesamte Zusammensetzung, enthalten sein.

Geeignete sebumregulierende Wirkstoffe können ausgewählt sein aus Acnacidol, Azelainsäure, Azeloglycina und einem Extrakt aus Spiraea Ulmaria. Der Extrakt ist z.B. im Produkt Seburegul der Firma Silab enthalten. Die sebumregulierenden Wirkstoffe können in den erfindungsgemäßen Zusammensetzungen vorzugsweise in Mengen von 0,001 bis 5 Gew.-%, bevorzugt 0,01 bis 2 Gew.-% und besonders bevorzugt 0,1 bis 1 Gew.-%, jeweils bezogen auf die gesamte Zusammensetzung, enthalten sein.

In einer weiteren besonders bevorzugten Ausführungsform werden die Wirkstoffe (a) bis (e) in kosmetischen Hautbehandlungsmitteln verwendet, die in einem zuvor beschriebenen Träger bevorzugt mindestens zwei, mehr bevorzugt mindestens drei Wirkstoffe aus der zuvor genannten Gruppe enthalten. Insbesondere bevorzugt sind erfindungsgemäße Zusammensetzungen, die als weitere Wirkstoffe mindestens zwei Wirkstoffe aus der Gruppe der Vitamine (wie zuvor beschrieben), der Harnstoff-Derivate nach Formel (I), der natürlichen Betainverbindungen, der Xanthin-Derivate und/oder der hautberuhigenden Wirkstoffe enthalten.

Erfindungsgemäße Zusammensetzungen können als Pasten, Salben, Lotionen oder Cremes vorliegen. Feste Mittel können beispielsweise als loser Puder, gepresster Puder oder als Stift vorliegen.

Vorteilhafterweise liegen erfindungsgemäße Zusammensetzungen in Form einer flüssigen, fliessfähigen oder festen Öl-in-Wasser-Emulsion, Wasser-in-Öl-Emulsion, Mehrfach-Emulsion, insbesondere einer Öl-in-Wasser-in-Öl- oder Wasser-in-Öl-in-Wasser-Emulsion, Makroemulsion, Miniemulsion, Mikroemulsion, PIT-Emulsion, Nanoemulsion, Pickering-Emulsion, Hydrodispersion, eines Hydrogels, eines Lipogels, einer ein- oder mehrphasigen Lösung, eines Schaumes, eines Puders oder einer Mischung mit mindestens einem als medizinischen Klebstoff geeigneten Polymer vor. Die Mittel können auch in wasserfreier Form, wie beispielsweise einem Öl oder einem Balsam, dargereicht werden. Hierbei kann der Träger ein pflanzliches oder tierisches Öl, ein Mineralöl, ein synthetisches Öl oder eine Mischung solcher Öle sein.

Besonders bevorzugte erfindungsgemäße Zusammensetzungen liegen in Form einer fließfähigen Emulsion vor, die topisch auf die Haut und/oder die Hautanhangsgebilde aufgebracht werden kann.

Geeignete Emulsionen im Sinne der Erfindung enthalten bevorzugt mindestens einen weiteren hautkonditionierenden Wirkstoff und mindestens einen Emulgator.

Unter geeigneten konditionierenden Wirkstoffen sind bevorzugt solche Substanzen zu verstehen, die auf keratinische Materialien, insbesondere auf die Haut, aufziehen und die physikalischen und sensorischen Eigenschaften sowohl der Haut als auch des Produktes als solchem verbessern. Konditionierungsmittel glätten die oberste Schicht der Haut und machen sie weich und geschmeidig. Über die Auswahl der Konditionierungsmittel (fettig - weniger fettig, schnell oder langsam spreitend, schnell oder langsam in die Haut einziehend und so weiter) lässt sich das Hautgefühl des gesamten Produktes einstellen. Bevorzugte konditionierende Wirkstoffe können ausgewählt sein aus Fettstoffen, insbesondere pflanzlichen Ölen, wie Sonnenblumenöl, Olivenöl, Sojaöl, Rapsöl, Mandelöl, Jojobaöl, Orangenöl, Weizenkeimöl, Pfirsichkernöl und den flüssigen Anteilen des Kokosöls, Lanolin und seinen Derivaten, flüssigen Paraffinölen, Isoparaffinölen und synthetischen Kohlenwasserstoffen, Di-n-alkylethern mit insgesamt 12 bis 36 C-Atomen, z. B. Din-octylether und n-Hexyl-n-octylether, Fettsaeuren, besonders linearen und/oder verzweigten, gesaettigten und/oder ungesaettigten C₈₋₃₀-Fettsaeuren, Fettalkoholen, besonders gesättigten, ein- oder mehrfach ungesättigten, verzweigten oder unverzweigten Fettalkoholen mit 4-30 Kohlenstoffatomen, die mit 1-75, bevorzugt 5-20 Ethylenoxid-Einheiten ethoxyliert und/oder mit 3-30, bevorzugt 9-14 Propylenoxid-Einheiten propoxyliert sein können, Esterölen, das heißt Estern von C₆₋₃₀-Fettsaeuren mit C₂₋₃₀-Fettalkoholen, Hydroxycarbonsäurealkylestern, Dicarbonsäureestern wie Di-n-butyladipat sowie Diolestern wie Ethylenglykoldioleat oder Propylenglykoldi(2-ethylhexanoat), symmetrischen, unsymmetrischen oder cyclischen Estern der Kohlensäure mit Fettalkoholen, z. B. Glycerincarbonat oder Dicaprylylcarbonat (Cetiol^{®} CC), Mono,- Di- und Trifettsaeureestern von gesättigten und/oder ungesättigten linearen und/oder verzweigten Fettsäuren mit Glycerin, die mit 1-10, bevorzugt 7- 9 Ethylenoxid-Einheiten ethoxyliert sein können, z. B. PEG-7 Glyceryl Cocoate, Wachsen, insbesondere Insektenwachsen, Pflanzenwachsen, Fruchtwachsen, Ozokerit, Mikrowachsen, Ceresin, Paraffinwachsen, Triglyceriden gesaettigter und gegebenenfalls hydroxylierter C₁₆₋₃₀-Fettsaeuren, z. B. gehärteten Triglyceridfetten, Phospholipiden, beispielsweise Sojalecithin, Ei-Lecithin und Kephalinen, Shea-Butter, Siliconverbindungen, ausgewählt aus Decamethylcyclopentasiloxan, Dodecamethylcyclohexasiloxan und Siliconpolymeren, die gewünschtenfalls quervernetzt sein können, z. B. Polydialkylsiloxanen, Polyalkylarylsiloxanen, ethoxylierten und/oder propoxylierten Polydialkylsiloxanen mit der früheren INCI-Bezeichnung Dimethicone Copolyol, sowie Polydialkylsiloxanen, die Amin-und/oder Hydroxy-Gruppen enthalten, bevorzugt Substanzen mit den INCI-Bezeichnungen Dimethiconol, Amodimethicone oder Trimethylsilylamodimethicone. Die Einsatzmenge der Fettstoffe in den erfindungsgemäßen Zusammensetzungen beträgt bevorzugt 0,1-99 Gew.-%, besonders bevorzugt 2-50 Gew.-% und außerordentlich bevorzugt 5-20 Gew.-%, jeweils bezogen auf die gesamte Zusammensetzung.

Geeignete oberflächenaktive Substanzen und/oder Emulgatoren sind beispielsweise Anlagerungsprodukte von 4 bis 30 Mol Ethylenoxid und/oder 0 bis 5 Mol Propylenoxid an lineare C₈-C₂₂-Fettalkohole, an C₁₂-C₂₂-Fettsaeuren und an C₈-C₁₅-Alkylphenole, C₁₂-C₂₂-Fettsaeuremono- und -diester von Anlagerungsprodukten von 1 bis 30 Mol Ethylenoxid an C₃-C₆-Polyole, insbesondere an Glycerin, Ethylenoxid- und Polyglycerin-Anlagerungsprodukte an Methylglucosid-Fettsäureester, Fettsäurealkanolamide und Fettsäureglucamide, C₈-C₂₂-Alkylmono- und -oligoglycoside und deren ethoxylierte Analoga, wobei Oligomerisierungsgrade von 1,1 bis 5, insbesondere 1,2 bis 2,0, und Glucose als Zuckerkomponente bevorzugt sind, Gemische aus Alkyl-(oligo)-glucosiden und Fettalkoholen, z. B. das im Handel erhältliche Produkt Montanov^{®} 68, Anlagerungsprodukte von 5 bis 60 Mol Ethylenoxid an Rizinusöl und gehärtetes Rizinusöl, Partialester von Polyolen mit 3-6 Kohlenstoffatomen mit gesättigten C₈-C₂₂-Fettsaeuren, Sterole (Sterine), insbesondere Cholesterol, Lanosterol, Beta-Sitosterol, Stigmasterol, Campesterol und Ergosterol sowie Mykosterole, Phospholipide, vor allem Glucose-Phospolipide, Fettsaeureester von Zuckern und Zuckeralkoholen wie Sorbit, Polyglycerine und Polyglycerinderivate, bevorzugt Polyglyceryl-2-dipolyhydroxystearat (Handelsprodukt Dehymuls^{®} PGPH) und Polyglyceryl-3-diisostearat (Handelsprodukt Lameform^{®} TGI) sowie lineare und verzweigte C₈-C₃₀-Fettsaeuren und deren Na-, K-, Ammonium-, Ca-, Mg-und Zn-Salze.

Die erfindungsgemäßen Zusammensetzungen können die Emulgatoren bevorzugt in Mengen von 0,1 bis 25 Gew.-%, besonders bevorzugt 0,5-15 Gew.-%, bezogen auf die gesamte Zusammensetzung, enthalten.

Weitere geeignete Zusatzstoffe der Emulsionen sind Verdickungsmittel, z. B. anionische Polymere aus Acrylsäure, Methacrylsäure, Crotonsäure, Maleinsäureanhydrid und 2-Acrylamido-2-methylpropansulfonsaeure, wobei die sauren Gruppen ganz oder teilweise als Natrium-, Kalium-, Ammonium-, Mono- oder Triethanolammonium-Salz vorliegen können und wobei mindestens ein nichtionisches Monomer enthalten sein kann. Bevorzugte nichtionogene Monomere sind Acrylamid, Methacrylamid, Acrylsäureester, Methacrylsäureester, Vinylpyrrolidon, Vinylether und Vinylester. Bevorzugte anionische Copolymere sind Acrylsäeure-Acrylamid-Copolymere sowie insbesondere Polyacrylamidcopolymere mit Sulfonsäuregruppen-haltigen Monomeren. Diese Copolymere können auch vernetzt vorliegen. Geeignete Handelsprodukte sind Sepigel^{®}305, Simulgel^{®} 600, Simulgel^{®} NS und Simulgel^{®} EPG der Firma SEPPIC. Weitere besonders bevorzugte anionische Homo- und Copolymere sind unvernetzte und vernetzte Polyacrylsäuren. Solche Verbindungen sind zum Beispiel die Handelsprodukte Carbopol^{®}. Ein besonders bevorzugtes anionisches Copolymer enthält als Monomer zu 80-98% eine ungesättigte, gewünschtenfalls substituierte C₃₋₆-Carbonsaeure oder ihr Anhydrid sowie zu 2-20% gewünschtenfalls substituierte Acrylsäureester von gesättigten C₁₀₋₃₀-Carbonsaeuren, wobei das Copolymer mit den vorgenannten Vernetzungsagentien vernetzt sein kann. Entsprechende Handelsprodukte sind Pemulen^{®} und die Carbopol^{®}-Typen 954, 980, 1342 und ETD 2020 (ex B.F. Goodrich).

Weitere geeignete Zusatzstoffe sind beispielsweise:
- Parfumöle,
- Substanzen zur Einstellung des pH-Wertes,
- Komplexbildner wie EDTA, NTA, β-Alanindiessigsaeure und Phosphonsaeuren,
- Treibmittel wie Propan-Butan-Gemische, Pentan, Isopentan, Isobutan, N₂O, Dimethylether, CO₂ und Luft,
- Pigmente und/oder
- Abrasivstoffe.

Die erfindungsgemäßen Zusammensetzungen weisen hervorragende Eigenschaften in der Anwendung auf der Haut auf.

Regelmäßig angewendet (beispielsweise zweimal täglich) reduzieren sie Hautfalten und/oder - fältchen bereits ab einer Anwendungsdauer von wenigen Wochen.

So konnte gezeigt werden, dass zwei von drei Falten in ihrer mittleren Tiefe signifikant reduziert werden konnten.

Darüber hinaus konnte die Hautfeuchtigkeit schlaffer und/oder müder Haut durch die Anwendung der erfindungsgemäßen Zusammensetzungen signifikant verbessert werden.

Ein zweiter Gegenstand der Erfindung ist daher die kosmetische, nicht-therapeutische Verwendung einer erfindungsgemäßen Zusammensetzung zur Minimierung von Hautfalten und - fältchen und/oder zur Behandlung der Anzeichen müder und/oder schlaffer Haut.

### Beispiele:

### 1) Ausführungsbeispiele für erfindunpspemäße Zusammensetzungen (Gesichtscremes 1 und 2)

| | Beispiel 1 [Gew.-%] | Beispiel 2 [Gew.-%] |
|---|---|---|
| Montanov 202^{®1} | 5 | 5 |
| Capryl-/Caprinsäure Triglycerid | 7 | 7 |
| Cetiol CC^{®2} | 5 | 5 |
| Novata AB PH^{®3} | 2 | 2 |
| DC EL-8040 ID^{®4} | 0,9 | 1 |
| Tocopherolacetat | 0,5 | 0,3 |
| Cetiol SB 45^{®5} | 1,5 | 2 |
| Coffein | 0,3 | |
| Glycerin | 5 | 5 |
| Hexandiol-1,6 | 5 | 5 |
| Betafin BP 20^{®6} | 2 | 2 |
| Tego Carbomer 140^{®7} | 0,4 | 0,35 |
| Hydrovance^{®8} | | 3 |
| Climbazol | 0,05 | 0,075 |
| Panthenol 75% | 0,75 | |
| Bisabolol | | 0,05 |
| Matrixyl 3000^{®9} | 3 | 3 |
| Simulgel EPG^{®10} | 1 | 1 |
| Hyaluronic Filling Sheres^{®11} | 1 | 1 |
| Plantactiv AGL^{®12} | 0,1 | 0,2 |
| Parfum, Konservierungsmittel | q.s. | q.s. |
| Wasser | ad 100 | ad 100 |

In der Tabelle wurden die folgenden Handelsprodukte eingesetzt:
1 INCI-Bezeichnung: Arachidyl Alcohol, Behenyl Alcohol, Arachidyl Glucoside; Seppic
2 INCI-Bezeichnung: Dicaprylyl Carbonate; BASF
3 INCI-Bezeichnung: Coco Glycerides; BASF
4 INCI-Bezeichnung: Isododecane, Dimethicone Crosspolymer; Dow Corning
5 INCI-Bezeichnung: Shea Butter (Butyrospermum Parkii); BASF
6 INCI-Bezeichnung: Trimethylglycine; Finnfeeds Finnland Oy
7 INCI-Bezeichnung: Carbomer; Evonik
8 INCI-Bezeichnung: Hydroxyethyl Urea; Akzo Nobel
9 INCI-Bezeichnung: Glycerin, Aqua (Water), Butylene Glycol, Carbomer, Coco Glucoside, Palmitoyl Oligopeptide, Palmitoyl Tetrapeptide-7; Sederma,
10 INCI-Bezeichnung: Sodium Acrylate/Sodium Acryloyldimethyl Taurate Copolymerf, Polyisobutene, Caprylyl/Capryl Glucoside; Seppic
11 INCI-Bezeichnung: Ethylhexyl Palmitate, Silica Dimethyl Silylate, Butylene Glycol, Hyaluronic Acid; BASF
12 INCI-Bezeichnung: Ammonium Glycyrrhizate; BASF

### 2) Wirkungsnachweise

Die Zusammensetzung 1 der Tabelle wurde von 31 weiblichen Probanden im Alter von 54 ± 8 Jahren für 4 Wochen getestet.

Am ersten Tag verbrachten die Probanden 30 Minuten in einem klimatisierten Raum (21 ± 1°C und 50 ± 5% relative Luftfeuchtigkeit).

Anschließend wurde die Haut der Probanden (Haut auf den Wangenknochen) dreidimensional vermessen (DermaTOP).

Die Hautfeuchtigkeit der betreffenden Hautregion wurde bei jedem Probanden zudem mit einem Corneometer gemessen.

Anschließend wurde die Tagescreme 1 unter Aufsicht und Anleitung eines technischen Mitarbeiters auf die gekennzeichneten Hautpartien eines jeden Probanden (s.o.) aufgebracht, und die Probanden wurden instruiert, die Creme für einen Zeitraum von vier Wochen zweimal täglich auf die gekennzeichneten Hautpartien aufzutragen.

Nach 28 Tagen kamen die Probanden zur erneuten Vermessung der Haut, jeweils 10 bis 16 Stunden nach der letzten Produktapplikation.

Die jeweiligen o.g. Messungen wurden wiederum durchgeführt, nachdem die Probanden 30 Minuten in einem klimatisierten Raum (21 ± 1°C und 50 ± 5% relative Luftfeuchtigkeit) verbracht hatten.

Ergebnis der Messungen war, dass zwei von drei Falten (66,7%) in ihrem Volumen und ihrer mittleren Tiefe reduziert werden konnten (als Vergleich dienten unbehandelte, benachbarte Hautpartien).

Die Corneometer-Messungen ergaben, dass die Hautfeuchtigkeit der mit der erfindungsgemäßen Creme 1 behandelten Hautpartien (s.o.) signifikant verbessert werden konnte, während unbehandelte, benachbarte Hautpartien einen leichten Hautfeuchtigkeitsverlust zeigten.

## Patentansprüche

1. Kosmetische Zusammensetzung, enthaltend in einem geeigneten kosmetischen oder dermatologischen Träger
(a) Climbazol und/oder eines seiner physiologisch verträglichen Salze,
(b) Tocopherol und/oder ein(e) physiologisch verträgliche(s) Salz,
(c) Glycyrrhizinsäure und/oder ein physiologisch verträgliches Salz,
(d) Hyaluronsäure und/oder ein physiologisch verträgliches Salz und
(e) mindestens ein Polyol.

2. Kosmetische Zusammensetzung nach Anspruch 1, **dadurch gekennzeichnet, dass** sie alpha-Tocopherol und/oder alpha-Tocopherolacetat enthält.

3. Kosmetische Zusammensetzung nach einem der Ansprüche 1 oder 2, **dadurch gekennzeichnet, dass** sie mindestens ein Polyol, ausgewählt aus 1,2-Propylenglycol, Glycerin, Diglycerin, Triglycerin, 1,2-Butandiol, 1,3-Butandiol, 1,2-Pentandiol, 1,5-Pentandiol, 1,2-Hexandiol, 1,6-Hexandiol, Sorbitol und/oder Sorbitan, enthält.

4. Kosmetische Zusammensetzung nach einem der Ansprüche 1 bis 3, **dadurch gekennzeichnet, dass** sie - jeweils bezogen auf ihr Gesamtgewicht -
- 0,001 bis 2 Gew.-% mindestens einer Komponente (a),
- 0,01 bis 3 Gew.-% mindestens einer Komponente (b),
- 0,01 bis 5 Gew.-% mindestens einer Komponente (c),
- 0,001 bis 2 Gew.-% mindestens einer Komponente (d) und
- 1 bis 90 Gew.-% mindestens einer Komponente (e) enthält.

5. Kosmetische Zusammensetzung nach einem der Ansprüche 1 bis 4, **dadurch gekennzeichnet, dass** sie in Form einer Emulsion vorliegt, die topisch auf die Haut und/oder die Hautanhangsgebilde aufgebracht werden kann.

6. Kosmetische, nicht-therapeutische Verwendung einer Zusammensetzung nach einem der Ansprüche 1 bis 5 zur Minimierung von Hautfalten und -fältchen und/oder zur Behandlung der Anzeichen müder und/oder schlaffer Haut.

## Claims

1. A cosmetic composition, containing in a suitable cosmetic or dermatological carrier
a. climbazole and/or one of the physiologically acceptable salts thereof,
b. tocopherol and/or a physiologically acceptable salt,
c. glycyrrhizic acid and/or a physiologically acceptable salt,
d. hyaluronic acid and/or a physiologically acceptable salt, and
e. at least one polyol.

2. The cosmetic composition according to claim 1, **characterized in that** it contains alpha-tocopherol and/or alpha-tocopherol acetate.

3. The cosmetic composition according to one of claims 1 or 2, **characterized in that** it contains at least one polyol, selected from 1,2-propylene glycol, glycerol, diglycerol, triglycerol, 1,2-butanediol, 1,3-butanediol, 1,2-pentanediol, 1,5-pentanediol, 1,2-hexanediol, 1,6-hexanediol, sorbitol, and/or sorbitan.

4. The cosmetic composition according to one of claims 1 to 3, **characterized in that** it contains, based in each case on its total weight,
- 0.001 to 2% by weight of at least one component (a),
- 0.01 to 3% by weight of at least one component (b),
- 0.01 to 5% by weight of at least one component (c),
- 0.001 to 2% by weight of at least one component (d), and
- 1 to 90% by weight of at least one component (e).

5. The cosmetic composition according to one of claims 1 to 4, **characterized in that** it is present in the form of an emulsion that can be applied topically to the skin and/or skin appendages.

6. Cosmetic, nontherapeutic use of a composition according to one of claims 1 to 5 for minimizing skin folds and wrinkles and/or for treating signs of tired and/or sagging skin.

## Revendications

1. Composition cosmétique contenant dans un support cosmétique ou dermatologique approprié
(a) du climbazole et/ou un de ses sels physiologiquement acceptables,
(b) du tocophérol et/ou un sel physiologiquement acceptable,
(c) de l'acide glycyrrhizique et/ou un sel physiologiquement acceptable,
(d) de l'acide hyaluronique et/ou un sel physiologiquement acceptable et
(e) au moins un polyol.

2. Composition cosmétique selon la revendication 1, **caractérisée en ce qu'**elle contient de l'alpha-tocophérol et/ou de l'acétate d'alpha-tocophérol.

3. Composition cosmétique selon l'une des revendications 1 ou 2, **caractérisée en ce qu'**elle contient au moins un polyol choisi parmi le 1,2-propylène-glycol, le glycérol, le diglycérol, le triglycérol, le butane-1,2-diol, le butane-1,3-diol, le pentane-1,2-diol, le pentane-1,5-diol, l'hexane-1,2-diol, l'hexane-1,6-diol, le sorbitol et/ou le sorbitan.

4. Composition cosmétique selon l'une des revendications 1 à 3, **caractérisée en ce qu'**elle contient, à chaque fois par rapport à son poids total,
- 0,001 à 2% en poids d'au moins un composant (a),
- 0,01 à 3% en poids d'au moins un composant (b),
- 0,01 à 5% en poids d'au moins un composant (c),
- 0,001 à 2% en poids d'au moins un composant (d) et
- 1 à 90% en poids d'au moins un composant (e).

5. Composition cosmétique selon l'une des revendications 1 à 4, **caractérisée en ce qu'**elle se présente sous la forme d'une émulsion qui peut être appliquée par voie topique sur la peau et/ou les phanères.

6. Utilisation non-thérapeutique cosmétique d'une composition selon l'une des revendications 1 à 5 pour réduire au minimum les rides et ridules de la peau et/ou pour traiter les signes d'une peau fatiguée et/ou flasque.
